# EUROPÄISCHE PATENTANMELDUNG

(11) **EP 3 524 197 A1**
(43) Veröffentlichungstag der Anmeldung: **14.08.2019**
(21) Anmeldenummer: 18155665.5
(22) Anmeldetag: 08.02.2018
(51) Int. Cl.: A61B 17/92

(54) **OPERATIONSHAMMER**

(71) Anmelder: Justus-Liebig-Universität Gießen, 35390 Gießen (DE)
(72) Erfinder: Jahnke, Alexander, 35392 Gießen (DE); Ishaque, Bernd, 35394 Gießen (DE); Rickert, Markus, 35398 Gießen (DE)
(74) Vertreter: Stumpf, Peter

(57) **Zusammenfassung**

Die Erfindung betrifft einen Operationshammer 1 zur Durchführung von Operationen bei gleichzeitiger Messung der dabei auftretenden Kräfte. Er umfasst einen Hammerkopf 2 zur Aufbringung einer Kraft auf eine Unterlage, einen Hammerstiel 3 zum Halten des Operationshammers 1, ein Krafterfassungsmittel 10 zur Erfassung der wirkenden Kräfte auf den Hammerkopf 2 in alle drei Raumrichtungen (x,y,z), wobei das Krafterfassungsmittel 10 so ausgebildet ist, dass es die erfassten Daten an ein Auswertemittel übertragen kann. Das Auswertemittel 30 ist im Operationshammer 1 integriert und so ausgebildet, dass es die erfassten Daten des Krafterfassungsmittels 10 auswerten und die Resultate an ein Ausgabemittel 40 übermitteln kann. Der Operationshammer 1 umfasst weiterhin ein Ausgabemittel 40 zur Ausgabe der Resultate des Auswertemittels 30. Zusätzlich umfasst der Operationshammer 1 ein Energiebereitstellungsmittel 50 zur Bereitstellung der notwendigen elektrischen Energie für das Krafterfassungsmittel 10, das Auswertemittel 30 und das Ausgabemittel 40. Dabei sind sowohl Krafterfassungsmittel 10, Auswertemittel 30, Ausgabemittel 40 als auch Energiebereitstellungsmittel 50 vollständig innerhalb des Operationshammers 1 angeordnet, so dass dieser sicher für Operationen einsetzbar und auch sterilisierbar ist.

## Beschreibung

Die vorliegende Erfindung betrifft einen Operationshammer mit integrierter Kraftmessung.

### Beschreibung und Einleitung des allgemeinen Gebietes der Erfindung

Operationshämmer werden üblicherweise bei der Durchführung von Operationen insbesondere im Bereich der Orthopädie verwendet. Dabei ist es von großer Bedeutung, dass deren Schläge mit der richtigen Kraft ausgeführt werden. Operationshämmer werden i.d.R. für das Einschlagen von Marknägeln, Raspeln, Endoprothesen und das Fügen von Köpfen von Hüftendoprothesen verwendet (jährlich ca. 250.000 primäre Hüftimplantationen, 160.000 Knieimplantationen [OECD-Datenbank von 2011]. Bei dem Fügen von Hüftendoprothesenköpfen auf den Prothesenkonus könnte eventuell eine Norm entwickelt werden, die eine intraoperative Fügekraft vorschreibt - bis dato gibt es hierfür allerdings kein Messinstrumentarium.

Deshalb wird der Krafteinsatz eines Operationshammers nicht sensorisch erfasst, sondern hängt vom Geschick und der Erfahrung des Chirurgen ab. Damit kann im Fall von Komplikationen aber auch nicht nachvollzogen werden, ob diese ihre Ursache in einem nicht optimal geführten Operationshammer haben könnten.

### Stand der Technik

Es sind Messhämmer bekannt, welche die Kraft mit der sie auf ein Werkstück einwirken erfassen können.

Ein solcher Messhammer ist beispielsweise in der Schrift EP 0 141 013 beschrieben worden. Dieser umfasst jedoch eine externe Stromversorgung und ist dadurch nicht sicher sterilisierbar. Weiterhin können die Anzeigeelemente aufgrund der Komplexität nicht in den Hammer integriert werden. Es wird ausdrücklich definiert, dass der Messhammer an einen externen Computer gekoppelt werden muss. Damit kann dieser Hammer nicht sterilisiert und damit nicht bei Operationen eingesetzt werden.

Die Schrift DE 199 06 140 beschreibt ein Messsystem zur Untersuchung von motorischen Reflexen. Dieses System umfasst auch einen Reflexhammer, bei welchem Zeitpunkt, Stärke und auch Dauer des Hammerschlages registriert werden. Dieser umfasst jedoch auch eine externe Stromversorgung und ist dadurch nicht sicher sterilisierbar. Außerdem verfügt der hierbei verwendete Hammer nur über eine 1-Achs-Sensorik. Bei nicht orthogonaler bzw. planarer Impulseinleitung, wie sie bei Operationen häufig vorkommen, werden Querkräfte verursacht, die mit einer 1-Achs-Sensorik nicht berücksichtigt werden können.

### Aufgabe

Aufgabe der vorliegenden Erfindung ist es einen Hammer mit integrierter Kraftmessung bereitzustellen, welcher bei Operationen sicher eingesetzt werden kann.

### Lösung der Aufgabe

Die Aufgabe wird durch die Vorrichtung mit den Merkmalen des Hauptanspruchs gelöst; vorteilhafte Weiterbildungen der Erfindung sind in den Unteransprüchen beschrieben.

Es handelt sich um einen instrumentierten und hermetisch versiegelbaren Operationshammer, der zur Dokumentation von intraoperativ auftretenden Einschlagskräften als auch der Fügekräfte von Hüftköpfen auf den Konus der jeweiligen Prothese (z.B. bei der Implantation von Endoprothesen) Verwendung finden kann.

Der erfindungsgemäße Operationshammer 1 umfasst einen Hammerkopf 2 zur Aufbringung einer Kraft auf eine Unterlage, einen Hammerstiel 3 zum Halten des Operationshammers 1. Der Hammerkopf 2 als auch der Hammerstiel 3 bestehen vorzugsweise aus verschweißbarem Edelstahl (z.B. Edelstahl gemäß DIN 1.4035) damit eine hermetische Versiegelung realisierbar ist. Der Hammerkopf 3 kann hierbei einstückig als auch mehrstückig ausgebildet sein. In einer mehrstückigen Ausführungsform kann der Hammerkopf 2 aus verschiedenen Materialien bestehen. Dabei kann der Hammerkopf 2 eine Schlagfläche mit auswechselbaren Kunststoffbacken beispielsweise aus PE oder Silikon aufweisen. Die Anforderungen an Operationswerkzeuge sind dabei durch die länderspezifischen Regularien festgelegt. Der Hammerstiel 3 weist vorzugsweise eine geriffelte bzw. ergonomische Struktur für einen sicheren Griff auf. Dabei kann der Hammerstiel 3 zur Gewichtsersparnis sterilisierbare Kunststoffblenden aufweisen. Diese bestehen beispielsweise aus Polyetheretherketon oder PEEK. Der Hammerkopf 2 weist je nach Einsatzgebiet eine Masse m zwischen 500g und 1500g auf. Beispielhafte Gewichtskategorien sind z.B. kleiner Hammerkopf m = 500 g; großer Hammerkopf m = 920 g. Hierbei beträgt bevorzugt der minimale Durchmesser des Hammerkopfes 2 ca. 40 mm und der minimale Durchmesser des Hammerstiles 3 ca. 12 mm. Der Hammerstiel 2 weist in einer bevorzugten Ausführungsform einen abgesetzten Hammergriff aus. Dieser Hammergriff weist einen größeren Durchmesser als der übrige Hammerstiel 2 auf, um diesen besser greifen zu können. Der Hammerstiel 3 kann hierbei einstückig als auch mehrstückig ausgebildet sein. In einer mehrstückigen Ausführungsform kann der Hammerstiel 3 aus verschiedenen Materialien bestehen. Dabei kann der Hammerstiel 3 eine oder mehrere Griffflächen mit auswechselbaren Kunststoffbacken beispielsweise aus PEEK, PE oder Silikon aufweisen. Der Durchmesser dieses Hammergriffs beträgt wenigstens 20 mm um einen ergonomischen Griff zu realisieren, die Stabilität des Hammers zu minimieren und die Unterbringung der Elektronik zu gewährleisten

Weiterhin umfasst der erfindungsgemäße Operationshammer 1 ein Krafterfassungsmittel 10. Dieses ist vorzugsweise ein 3-Achsen-Beschleunigungssensor zur Erfassung der Beschleunigung des Hammerkopfes in alle drei Raumrichtungen (x,y,z). Aus der Masse des Hammers kann aus den Beschleunigungen direkt die Kraft bestimmt werden. Mit einem 3-Achsen-Beschleunigungssensor lässt sich die resultierende Kraft bei nicht orthogonal eingeleitetem Impuls berechnen und somit die resultierende, axial wirkende Kraft bestimmen.

Bei diesem Beschleunigungssensor handelt es sich um einen 3-Achsen-Beschleunigungssensor. Beschleunigungssensoren messen üblicherweise die Beschleunigung, indem die auf eine Testmasse wirkende Trägheitskraft bestimmt wird. Somit kann z. B. bestimmt werden, ob eine Geschwindigkeitszunahme oder - abnahme stattfindet.

Als Beschleunigungssensor kann beispielsweise ein induktiver Beschleunigungssensor verwendet werden. Bei der Bewegung des Hammerkopfes wird durch einen Permanentmagneten in einer Spule eine elektrische Spannung induziert. Dieses Wirkprinzip ist ähnlich wie in einer Schüttellampe. In einer Ausführungsform des erfindungsgemäßen Operationshammers 1 ist der Beschleunigungssensor des Krafterfassungsmittels 10 als ein induktiver Beschleunigungssensor ausgebildet ist, welcher so ausgebildet ist, dass bei der Bewegung des Hammerkopfes 2 eine elektrische Spannung induziert wird, deren Stärke gemessen werden kann.

Als Beschleunigungssensor kann alternativ ein piezoelektrischer Beschleunigungssensor verwendet werden.

In einer alternativen Ausführungsform des erfindungsgemäßen Operationshammers 1 ist der Beschleunigungssensor des Krafterfassungsmittels 10 als ein piezoelektrischer Beschleunigungssensor ausgebildet, welcher so ausgebildet ist, dass die bei der Bewegung des Hammerkopfes 2 auftretenden dynamischen Druckschwankungen auf ein piezoelektrisches Element in elektrische Signale umgesetzt werden und, deren Stärke gemessen werden kann. Diese Druckschwankungen werden durch das Auftreffen des Hammerkopfes auf eine Unterlage (z.B. eine zu implantierende Hüftprothese) hervorgerufen und wirken bei einer Beschleunigung des Gesamtsystems auf die Piezokeramik, welche im Hammerkopf angeordnet ist.

Weiterhin umfasst der Operationshammer 1 ein Auswertemittel 30, welches in den Operationshammer 1 integriert ist. Bevorzugt ist das Auswertemittel 30 im Hammerstiel angeordnet. Als Auswertemittel dient dabei ein Gerät zur elektronischen Datenverarbeitung z.B. ein in den Hammerkopf 2 integrierter programmierbarer Mikroprozessor. Das Auswertemittel 30 ist weiterhin so ausgebildet, dass es die Ergebnisse des Krafterfassungsmittels 10 auswerten und die Resultate an ein Ausgabemittel 40 übermitteln kann.

Die durch das Krafterfassungsmittel 10 ermittelten Messwerte werden dabei durch das Auswertemittel 30 mit Kraftnormwerten für die entsprechenden Parameter verglichen, um eine Einschätzung der durch den Operationshammer aufgebrachten Kraft zu erhalten. Das Krafterfassungsmittel 10 ist dabei so ausgebildet, dass es die erfassten Daten an ein Auswertemittel 30 übertragen kann.

Die Datenübertragung kann über eine feste Datenleitung oder drahtlos beispielsweise über eine Funkverbindung erfolgen. Da sowohl Krafterfassungsmittel 10 als auch Auswertemittel 30 im Operationshammer 1 angeordnet sind, ist eine drahtgebundene Übertragung zu bevorzugen.

Die Kraftnormwerte sind dabei in einer internen Datenbank des Auswertemittels 30 hinterlegt oder werden durch einen Datenaustausch mit einer externen Datenbank beispielsweise über ein Netzwerk durch das Auswertemittel 30 bezogen. Dazu weist das Auswertemittel 30 vorzugsweise eine Schnittstelle zum elektronischen Datenaustausch (engl. electronic data interchange, EDI) auf.

Weiterhin umfasst die Vorrichtung ein Ausgabemittel 40 zur Ausgabe des Ergebnisses des Auswertemittels 30. Die Ausgabe erfolgt dabei bevorzugt optisch und/oder akustisch. Als Ausgabemittel können dabei ein Lautsprecher und/oder ein Bildschirm oder eine LED dienen. Damit können sowohl die Messwerte der Kraftmessung nach der Operation ausgelesen werden, als auch die tatsächlichen Kräfte, die sich aus den Beschleunigungswerten in Abhängigkeit der Masse des Hammers ergeben über das Auswertemittel 30 (z.B. eine Induktionsschnittstelle mit programmierbarem Mikroprozessor) in Echtzeit berechnen werden. Diese Werte können dann an das Ausgabemittel 40 (z.B. LEDs (gelb = zu schwach, grün = optimal, rot = zu stark)) weitergegeben werden.

Das Ausgabemittel 40 ist geeignet, dem Operateur in Echtzeit anzuzeigen, ob er sich im Bereich risikobehafteter Schläge befindet, oder ob sich diese im akzeptablen Bereich befinden (z.B. beim Fügen des Hüftkopfes auf den Konus der Prothese).

Weiterhin umfasst der Operationshammer 1 ein Energiebereitstellungsmittel 50 zur Energiebereitstellung für das Auswertemittel 30 und das Ausgabemittel 40.

Eine erste Möglichkeit einer Energiebereitstellung ist die Nutzung der kinetischen Energie beim Beschleunigen des Hammers und beim letztendlichen Stoßimpuls. In einer bevorzugten Ausführungsform des erfindungsgemäßen Operationshammers 1, umfasst das Energiebereitstellungsmittel 50 hierbei einen Magnet und eine elektrische Spule. Dabei ist der Magnet in der Spule frei beweglich gelagert und im Inneren des Hammerkopfes so angeordnet, dass er sich beim Bewegen des Hammers innerhalb der Spule verschieben kann. Diese Verschiebung führt zur Erzeugung einer induktiven Spannung, die letztendlich die Versorgungspannung für die übrigen Bauelemente im Inneren des Hammers realisiert. Gleichzeitig wird die induzierte Spannung in Kondensatoren gespeichert um eine permanente Spannungsversorgung zu gewährleisten.

Ergänzend dazu weist die Vorrichtung vorzugsweise noch Schnittstellen zum Datenaustausch zwischen dem Krafterfassungsmittel 10, dem Auswertemittel 30 und dem Ausgabemittel 40 auf. Die Datenübertragung kann über eine feste Datenleitung oder drahtlos (z.B. über Funk) erfolgen.

Dabei ist es so, dass sowohl Krafterfassungsmittel 10, Auswertemittel 30, Ausgabemittel 40 als auch Energiebereitstellungsmittel 50 vollständig innerhalb des Operationshammers 1 angeordnet sind, so dass dieser sicher für Operationen einsetzbar und auch sterilisierbar ist.

In einer weiteren Ausführungsform umfasst der erfindungsgemäße Operationshammer 1 weiterhin ein Eingabemittel 20 zur Eingabe der für die Einstufung der Kraft notwendigen Parameter. Diese Parameter können dabei sein: Art der Operation, Typ und Material des Implantats/der Prothese, Alter und Geschlecht der zu behandelnden Person.

Sowohl das Krafterfassungsmittel 10 als auch das Eingabemittel 20 sind dabei so ausgebildet, dass sie die erfassten Daten des Krafterfassungsmittels 10 bzw. die eingegebenen Parameter an ein Auswertemittel 30 übertragen können.

Die Datenübertragung kann über eine feste Datenleitung oder drahtlos beispielsweise über eine Funkverbindung erfolgen.

In einer alternativen Ausführungsform des erfindungsbemäßen Operationshammers umfasst dieser weiterhin noch eine Ladestation 60.

Diese Ladestation 60 ist so ausgebildet, dass über diese Ladestation die gespeicherten Daten des Krafterfassungsmittels 10 und die durch das Auswertemittel 30 direkt intraoperativ erzeugten Schlagkräfte, z.B. ob der Konus mit einer ausreichenden Kraft gefügt wurde, dokumentiert und ausgelesen werden können. Während der dynamischen Nutzung des Hammers 1 wird die Versorgungsspannung, wie bereits oben beschrieben, gewährleistet.

Weiterhin ist die Ladestation 60 bevorzugt so ausgebildet, dass der Operationshammer 1 mit der Ladestation 60 Daten austauschen kann, womit die Ladestation 60 als Auswertemittel 30 und/oder als Eingabemittel 20 dienen kann.

Um einen direkten Einsatz nach der Sterilisation des Hammers zu gewährleisten, soll durch Kondensatoren eine Grundkapazität sichergestellt werden, z.B. durch eine ebenfalls sterilisierbare Ladestation im OP-Bereich kurz vorab der Nutzung. Die komplett integrierte Messkette als auch das Ausgabeelement und die interne Speicherung der Messdaten erlauben die Dokumentation der auftretenden Schlagkräfte. Ferner können die Schlagkräfte in Echtzeit unter Verwendung der integrierten Messkette interpretiert und optisch wiedergegeben werden. Es sind keine Anschlusselemente an den Hammer während des Betriebs notwendig. Sowohl der Export der Daten als auch die Gewährleistung der "Startkapazität" erfolgt vorzugsweise durch eine sterilisierbare Ladestation unter Verwendung einer induktiven Schnittstelle.

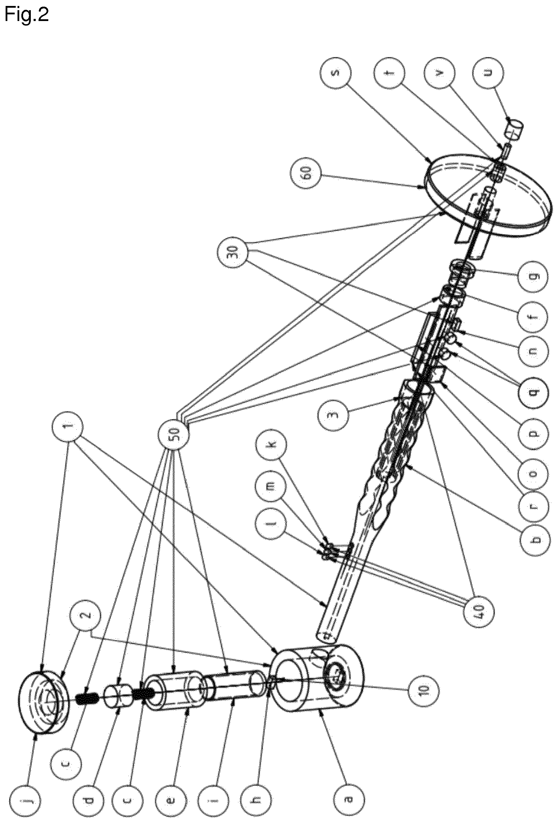

Eine beispielhafte Ausführungsform des Operationshammers 1 zeigt die Abbildung Fig.2 in einer Explosionszeichnung.

Der Hammerkopf 2 umfasst dabei einen Hammerkopfmantel a der die anderen Bauteile des Hammerkopfes umgibt.

Innerhalb des Hammerkopfmantels a befinden sich ein erster Magnet d, Federn c, eine Ladespule e ein zweiter Magnet i und ein Beschleunigungssensor h.

Der erste Magnet d ist innerhalb einer Kunststoffröhre i angeordnet, wobei diese Kunststoffröhre i sich ihrerseits in einer Ladespule e befindet und röhrenförmig ausgebildet ist. Dabei ist der erste Magnet d so angeordnet, dass er sich parallel zur Spule e bewegen kann. Dadurch wird ein Strom induziert der zu Energieversorgung des Beschleunigungssensors h genutzt werden kann. Deshalb dienen der erste Magnet d mit den Federn c, der Spule e und der Kunststoffröhre i als Energiebereitstellungsmittel 50.

Weiterhin umfasst der Hammerkopf 2 einen Verschluss j. Dieser dient dazu Bauteile in den Hammerkopf 2 einsetzen zu können.

Der Hammerstiel 2 umfasst das Ausgabemittel 40. Das Ausgabemittel 40 wiederum umfasst in dieser Ausführungsform hier eine grüne LED k eine gelbe LED m und eine rote LED I.

Zusätzlich weist der Hammerstiel 3 eine Platine r mit einem AV-Wandler n, einem Mikroprozessor o, einem Datalogger p und Kondensator q auf. Dabei dienen der AV-Wandler n, Mikroprozessor o und Datalogger p als Auswerteinheit 30.

Der AV-Wandler n dient zur Umwandlung der analogen Daten des Beschleunigungssensors h in digitale Daten. Der Mikroprozessor o dient zur Verarbeitung der digitalen Daten. Der Datalogger p dient zur Zwischenspeicherung der Daten. Die Kondensatoren q dienen hier als Energiebereitstellungsmittel 50 für das Auswertemittel 30 und das Ausgabemittel 40. Weiterhin umfasst der Hammerstiel 3 eine Ladungsspule f. Diese ist so ausgebildet, dass sie durch Induktion Energie aufnehmen und die Kondensatoren q aufladen kann. Weiterhin umfasst der Hammerstiel 3 einen Verschluss g. Dieser dient dazu Bauteile in den Hammerstiel 3 einsetzten zu können.

Weiterhin umfasst der Hammerstiel 3 einen Griff b. Dieser Griff weist eine geriffelte Oberfläche auf damit der Operationshammer 1 besser gegriffen werden kann.

Die Ladestation 60 umfasst ein Ladestationsgehäuse s zu Aufnahme der weiteren Bauteile der Ladestation 60. Das Ladestationsgehäuse s ist so ausgebildet, dass der Hammerstiel 3 in die Ladestation eingestellt werden kann. Weiterhin umfasst die Ladestation eine Spule t mit einem Eisenkern v. Dabei ist die Spule t so angeordnet, dass die an eine externe Wechselstromquelle anschließbar ist und über die Ladespule f die Kondensatoren q im Hammerstiel 3 mit Energie versorgen kann.

Alternativ ist der Eisenkern v beweglich innerhalb der Spule angeordnet. In diesem Fall kann das laden auch durch ein einfaches Schütteln des Operationshammers 1 mit der Ladestation 60 erfolgen. In beiden Fällen können die Spule t mit Eisenkern v als Energiebereitstellungsmittel 50 dienen.

Die Ladestation 60 ist hier so ausgebildet, dass sie Daten des Dataloggers p empfangen kann. Damit kann die Ladestation auch als Teil des Auswertemittels 30 dienen.

Weiterhin umfasst die Ladestation 60 einen Verschluss u. Dieser dient dazu Bauteile in das Ladestationsgehäuse s einsetzten zu können.

Die verschiedenen Ausführungsformen der Bauteile des erfindungsgemäßen Operationshammers 1 insbesondere der Beschleunigungssensoren für das Krafterfassungsmittel 10, des Auswertemittels 30, des Ausgabemittels 40 als auch des Energiebereitstellungsmittels 50 sind einzeln oder in Kombination einsetzbar.

Weiterhin kann jede dieser Ausführungsvarianten wenigstens eine sterilisierbare Kunststoffblende, ein Eingabemittel 20 und/oder oder eine Ladestation 60 umfassen.

Dabei sind auch diese Bauteile (sterilisierbare Kunststoffblende, Eingabemittel 20, Ladestation 60) unabhängig voneinander einzeln oder in Kombination implementierbar.

### Abbildungslegenden und Bezugszeichenliste

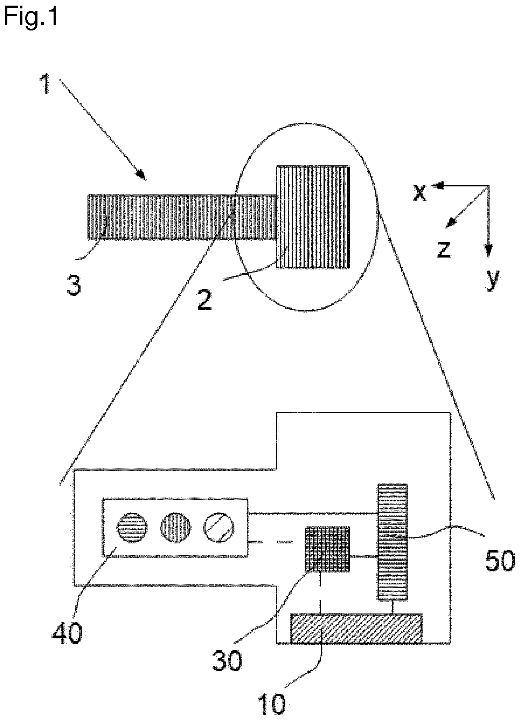

Fig. 1 schematische Darstellung eines erfindungsmäßen Operationshammers 1. Durchgezogene Linien zeigen dabei Wege der Energieübertragungen. Gestrichelte Linien zeigen Wege der Datenübertragung.
Fig.2 Explosionszeichnung einer beispielhaften Ausführungsform des erfindungsmäßen Operationshammers 1.

### Bezugszeichen

1 Operationshammer
2 Hammerkopf
3 Hammerstiel
10 Krafterfassungsmittel
20 Eingabemittel
30 Auswertemittel
40 Ausgabemittel
50 Energiebereitstellungsmittel
60 Ladestation

- a: Hammerkopfmantel
- b: Griff
- c: Feder
- d: Permanentmagnet
- e: Ladespule des Hammerkopfes
- f: Ladespule des Hammerstiels
- g: Verschluss des Hammerstiels
- h: Beschleunigungssensor
- i: Kunststoffröhre
- j: Verschluss Hammerkopf
- k: LED_grün
- l: LED_rot
- m: LED_gelb
- n: AV-Wandler
- o: Mikroprozessor
- p: Datalogger
- q: Kondensator
- r: Platine
- s: Ladestationsgehäuse
- t: Spule der Ladestation
- u: Verschluss der Ladestation
- v: Eisenkern

## Patentansprüche

1. Operationshammer (1) zur Verwendung bei von Operationen mit gleichzeitiger Messung der dabei auftretenden Kräfte umfassend
• einen Hammerkopf (2) zur Aufbringung einer Kraft auf eine Unterlage,
• einen Hammerstiel (3) zum Halten des Operationshammers (1),
• ein Krafterfassungsmittel (10) zur Erfassung der wirkenden Kräfte auf den Hammerkopf in alle drei Raumrichtungen (x,y,z) mittels wenigstens eines Beschleunigungssensors, wobei das Krafterfassungsmittel (10) dabei so ausgebildet ist, dass es die erfassten Daten auf ein Auswertemittel (30) übertragen kann.
• ein Auswertemittel (30), welches im Operationshammer (1) integriert ist und so ausgebildet ist, dass es die erfassten Daten des Krafterfassungsmittel (10) auswerten und die Resultate an ein Ausgabemittel (40) übermitteln kann,
• ein Ausgabemittel (40) zur Ausgabe der Resultate,
• ein Energiebereitstellungsmittel (50) zu Bereitstellung der notwendigen elektrischen Energie für das Krafterfassungsmittel (10), Auswertemittel (30) und Ausgabemittel (40),
**dadurch gekennzeichnet, dass** sowohl Krafterfassungsmittel (10), Auswertemittel (30), Ausgabemittel (40) als auch Energiebereitstellungsmittel (50) vollständig innerhalb des Operationshammer (1) angeordnet sind, so dass dieser, sicher für Operationen einsetzbar und auch sterilisierbar ist.

2. Operationshammer (1) gemäß Anspruch 1 **dadurch gekennzeichnet, dass** der der Hammerstiel (29 wenigstens eine sterilisierbare Kunststoffblende aufweist.

3. Operationshammer (1) gemäß Anspruch 1 oder 2 **dadurch gekennzeichnet, dass** der wenigstens eine Beschleunigungssensor des Krafterfassungsmittels (10) als ein induktiver Beschleunigungssensor ausgebildet ist, welcher so ausgebildet ist, dass bei der Bewegung des Hammerkopfes (2) eine elektrische Spannung induziert wird, deren Stärke gemessen werden kann.

4. Operationshammer (1) gemäß Anspruch 1 oder 2 **dadurch gekennzeichnet, dass** der wenigstens eine Beschleunigungssensor des Krafterfassungsmittels 10 als ein piezoelektrischer Beschleunigungssensor ausgebildet ist, welcher so ausgebildet ist, dass die bei der Bewegung des Hammerkopfes 2 auftretenden dynamische Druckschwankungen auf ein piezoelektrisches Element des piezoelektrischer Beschleunigungssensors in elektrische Signale umgesetzt werden, deren Stärke gemessen werden kann.

5. Operationshammer (1) gemäß einem der vorhergehenden Ansprüche **dadurch gekennzeichnet, dass** dieser weiterhin ein Eingabemittel (20) zur Eingabe und zur Übertragung an das Auswertemittel (30) der für die Einstufung der Kraft notwendigen Parameter aufweist.

6. Operationshammer (1) gemäß einem der vorhergehenden Ansprüche **dadurch gekennzeichnet, dass** dieser weiterhin eine Ladestation (60) aufweist, wobei diese so ausgebildet ist, dass über diese Ladestation die gespeicherten Daten des Krafterfassungsmittels (10) und die durch das Auswertemittel (30) direkt intraoperativ gemessenen Schlagkräfte, dokumentiert und ausgelesen werden können.

7. Operationshammer (1) gemäß einem der vorhergehenden Ansprüche **dadurch gekennzeichnet, dass** das Energiebereitstellungsmittel (50) einen Magnet und eine elektrische Spule umfasst, wobei der Magnet in der Spule frei beweglich gelagert und im Inneren des Hammerkopfes so angeordnet ist, dass er sich beim Bewegen des Hammers innerhalb der Spule verschieben kann.
